Europäisches Patentamt

European Patent Office

Office européen des brevets

(11) Publication number: **0 497 538 A2**

# EUROPEAN PATENT APPLICATION

(21) Application number: **92300682.9**

(22) Date of filing: **27.01.92**

(51) Int. Cl.5: **C07C 265/14**, C07C 263/20

(30) Priority: **28.01.91 US 647328**
**10.07.91 US 727707**

(43) Date of publication of application:
**05.08.92 Bulletin 92/32**

(84) Designated Contracting States:
**AT BE CH DE DK ES FR GB GR IT LI LU NL SE**

(71) Applicant: **THE DOW CHEMICAL COMPANY**
**2030 Dow Center Abbott Road**
**Midland, MI 48640(US)**

(72) Inventor: **Daussin, Rory D.**
**512 Chelsea**
**Bellaire, Texas 77401(US)**
Inventor: **Lowenkron, Steven B.**
**16207 LaCabana**
**Houston, Texas 77062(US)**
Inventor: **Nafziger, John L.**
**459 Southern Oaks**
**Lake Jackson, Texas 77566(US)**
Inventor: **Kent, Van A.**
**113 Cardinal**
**Lake Jackson, Texas 77566(US)**

(74) Representative: **Burford, Anthony Frederick et al**
**W.H. Beck, Greener & Co. 7 Stone Buildings**
**Lincoln's Inn**
**London WC2A 3SZ(GB)**

(54) **Process for reducing hydrolyzable chloride in toluene diisocyanate.**

(57) Hydrolyzable chloride in toluene diisocyanate (TDI), distillation bottoms is reduced by heating crude TDI, optionally in the presence of a solvent, to cause: (a) partial reflux, (b) partial reflux and fractionation, or (c) complete reflux and fractionation and/or by contacting toluene diisocyanate distillation residue with a material suitable for purging chloride-containing vapors under reaction conditions suitable to remove chloride-containing vapors from the toluene diisocyanate distillation residues. Viscosity-stable and reactive bottoms, from either process, can be reacted with compounds containing active hydrogen to form polyurethane, polyurea, and polyisocyanurate polymers, particularly foamed polymers.

EP 0 497 538 A2

This invention relates to a process for preparing reactive toluene diisocyanate bottoms. More particularly, this invention relates to reduction of hydrolyzable chloride in crude toluene diisocyanate and in toluene diisocyanate distillation bottoms.

The term "hydrolyzable chloride" refers to labile chlorine atoms which are free, ionically or covalently bonded within a compound, but have more ionic character than, for example, the chlorine atom present in chlorobenzene. Hydrolyzable chloride concentration can also be referred to as chloride equivalent or chloride level.

Toluene diisocyanates (hereinafter TDI) are commonly prepared by phosgenation of toluene diamines. After the phosgenation of toluene diamines to form TDI, the product diisocyanate is generally distilled from the reaction mixture in which it is prepared. This reaction product mixture, after phosgenation but prior to distillation of TDI monomer, shall hereafter be referred to as crude TDI. At the conclusion of the distillation, the residue of the distillation is commonly referred to as distillation bottoms or, more simply, just bottoms. The bottoms normally contain a quantity of high boiling residue composed of materials such as alpha and omega-isocyanato-biurets, polycarbodiimides, diisocyanatocarbodiimides, polyuretidinediones, isocyanurates and various other isocyanate adducts, including hydrolyzable chloride containing compounds. This residue is typically discarded.

The disposal of this residue is a serious problem to the TDI industry. It is costly and poses safety problems. The handling of this material is a problem because special equipment is needed to move and store it prior to disposal.

A number of processes for reduction of hydrolyzable chloride in isocyanates have been developed. These processes have been directed primarily to methylene diphenyl diisocyanate, but have been disclosed as being applicable to other materials as well. Some of the processes involve reduction of acidity levels. US-A-3,179,680 discloses that the concentration of hydrolyzable chloride in organic isocyanates can be reduced by heating the organic isocyanates in the presence of small amounts of water. However, water is reactive with isocyanates and can reduce yields or cause other processing problems. US-A-3,219,678 discloses that reduction of hydrolyzable chloride can be achieved by heating an isocyanate containing hydrolyzable chloride at temperatures considerably above those required and used for the cleavage of carbamoyl chlorides to form organic isocyanates and hydrogen chloride (hereinafter HCl), and then, passing an inert gas through the isocyanate to remove the HCl. This process, however, fails to address the problem of reformation of hydrolyzable chloride containing compounds. US-A-3,857,871 discloses that acidity and hydrolyzable chloride levels of polymethylene polyphenyl polyisocyanate (hereinafter PMDI) are reduced and reactivity increased by exposing the polyisocyanate in a liquid state at 350°F (177°C) to 450°F (232°C) to countercurrent treatment with an inert gas. However, use of heat and an inert gas alone does not appear to maximize removal of hydrolyzable chloride from crude TDI.

There have also been a number of treatment methods developed which include processes designed to convert bottoms to entirely new materials, processes to use them as fillers in various plastics materials, and processes designed to "purify" them. For example, US-A-4,506,040 discloses a process wherein TDI bottoms are reacted with an active hydrogen containing compound to form a polymer. The polymer is then dispersed in an organic solvent. A high molecular weight polyol is added and the solvent is removed, thereby producing a dispersion of the polymer in the high molecular weight polyol. The resulting dispersion is used to prepare polyurethane products, including foams and elastomers.

US-A-4,480,081 discloses a process wherein bottoms are reacted with a monohydroxylic compound, and the modified product is then reacted with a polyol. The final product is an isocyanate reactive compound for use in polyurethane formulations. US-A- 4,311,800 discloses a process wherein highly cross-linked insoluble bottoms are reacted with water and then alcohol to form soluble compounds containing hydroxyalkyl and urethane groups. They may also be recyclized by simple hydrolysis into toluene diamines (TDA), which is the starting material from which TDI is derived. US-A-4,489,177 discloses reaction of the TDI bottoms with a polyol to obtain a product having an OH-number of from 200 to 700. US-A-3,455,836 discloses addition of 4,4'-methylene bis(phenyl- isocyanate) to liquid TDI bottoms. The product can be reacted with active hydrogen containing materials.

DE-A-2,846,814 describes a process by which TDI bottoms are pulverized, treated with NCO- reactive compounds, and then dispersed in aliphatic polyols. The dispersions are used to manufacture urethane polymers. DE-A-2,942,678 discloses mixing ground bottoms with certain monohydric alcohols, diols, or triols to produce a mixture containing at least 2 percent hydroxyl groups; heating the mixture at greater than 130°C until homogeneous; and then cooling and grinding the product to use as a starting material in plastics. US-A-4,595,709 shows preparation of polyaddition products containing urethane groups by reacting the TDI distillation bottoms with a compound containing hydroxyl groups such that the NCO/OH equivalent ratio is less than 1.5:1. The product can be used to prepare flame-resistant polyurethanes. US-A-4,293,456 discloses use of bottoms directly as a reactive filler in polyurethanes to improve mechanical properties. US-A-4,251,401 discloses producing suspensions of the bottoms in polyhydroxyl compounds to use as the polyol component in forming polyurethanes. DE-A-2,846,815 discloses milling of TDI bottoms, followed by treatment with isocyanate-reactive compounds to prepare reactive materials which are useful as fillers or dispersants.

It would be desirable in the art to find a process to prepare TDI monomer wherein the distillation bottoms produced are relatively stable in viscosity and which also have a desirable reactivity when used to prepare a polyurethane product. Thus, it would be desirable to remove sufficient hydrolyzable chloride from crude TDI to produce both reduced hydrolyzable chloride TDI monomer and commercially useful distillation bottoms. It would also be desirable in the art to find a process to render conventional TDI distillation bottoms relatively stable in viscosity and desirably reactive when used to prepare a polyurethane product.

In one aspect, the invention is a process for reducing hydrolyzable chloride in crude TDI comprising heating crude TDI under conditions sufficient to (a) partially reflux, (b) partially reflux and fractionate, or (c) completely reflux and fractionate the crude TDI. The crude TDI can be admixed with a solvent or not. Heating the crude TDI serves to alter the equilibrium of the decomposition and reformation of hydrolyzable chloride containing compounds to promote decomposition of hydrolyzable chloride containing compounds into volatile chloride containing compounds. The partial or complete refluxing with fractionation, or partial refluxing alone, of the crude TDI serves to remove the volatile chloride containing compounds from the crude TDI.

Also an aspect of the present invention is a process for preparing viscosity-stable, reactive TDI bottoms comprising contacting TDI bottoms with a material suitable for purging chloride-containing vapors under reaction conditions suitable to remove chloride-containing vapors. In preferred embodiments the material is an aromatic solvent, a gas, or a combination thereof.

One utility of this invention is to produce low hydrolyzable chloride crude TDI which can be distilled to produce both low hydrolyzable chloride TDI monomer and also TDI distillation bottoms which are viscosity-stable and sufficiently reactive to be commercially useful. Another utility of the present invention is to reduce the hydrolyzable chloride concentration of TDI bottoms.

The present invention therefore provides processes for preparing treated TDI bottoms to improve their viscosity stability and also reactivity, such that they can be successfully used, either alone or blended with one or more other isocyanate products, as the isocyanate component in a polyurethane formulation. Stabilizing bottoms viscosity can result in increased shelf life for materials prepared with the viscosity-stable bottoms.

It is known in the art that at least some hydrolyzable chloride containing compounds, in crude TDI, will disassociate into HCl and isocyanates when heated. Reduction of hydrolyzable chloride in crude TDI can be achieved by decomposing hydrolyzable chloride containing compounds, in the crude TDI, into volatile chloride containing compounds and removing those volatile chloride containing compounds from the crude TDI. These volatile chloride containing compounds include at least HCl, but can also include other compounds. Discussion herein will be focussed on HCl, but it will be understood that the process of the present invention is applicable to all of these chloride containing compounds.

In the first aspect of the present invention, crude TDI is heated, with or without a solvent, sufficiently to cause partial reflux, partial reflux with fractionation, or complete reflux with fractionation. A liquid is completely refluxed by 1) heating the liquid to boiling, 2) passing the gases and vapor into a condenser, 3) condensing substantially all of the nongaseous components and 4) returning the condensate to the boiling liquid. Partial reflux of a liquid is achieved by the same procedure except that a portion of the vapor is released from the system.

The concept of chemical equilibrium is well known in the art. For the purposes of the present invention, stated briefly, equilibrium is the propensity of chemical compounds to exist not as pure materials, but rather as mixtures of products and their reactants. In other words, equilibrium is the state at which the rate of formation is equal to the rate of decomposition.

The process of this first aspect of the present invention involves affecting one and preferably two equilibria. One of these is the equilibrium of decomposition of hydrolyzable chloride containing compounds, in crude TDI, into HCl and isocyanates. The other is the solubility equilibrium of gaseous HCl in refluxing vapor.

Stated very generally, the process of this first aspect includes removing HCl from a crude TDI system, thereby reducing hydrolyzable chloride. Since HCl and hydrolyzable chloride containing compounds are in equilibrium, removing the HCl promotes further decomposition of hydrolyzable chloride containing compounds, thereby producing more HCl. Continuing this process for a sufficient time period produces a crude TDI with a relatively low concentration of hydrolyzable chloride. After distillation of TDI monomer, the distillation bottoms formed will preferably be desirably reactive and viscosity-stable.

However, before HCl can be removed from a crude TDI system and thereby affect the equilibrium of decomposition and reformation of hydrolyzable chloride containing compounds, there must be enough HCl present to be acted upon by a means of removal. At ambient temperatures, this equilibrium favors the production of hydrolyzable chloride containing compounds to such a great extent, that only a very small concentration of free HCl is present.

One part of the process of this first aspect incorporates, in general, applying heat to crude TDI to increase the equilibrium concentration of HCl. The preferred temperature range is from 40°C to 230°C, but preferably from 150°C to 200°C, and more preferably from 180°C to 195°C.

Another part of the process of this first aspect, applied concurrently with the heating aspect, incorporates increasing removal of HCl from crude TDI. This includes application of any one of three means to remove HCl from crude TDI. Those means are: 1) partial reflux, 2) partial reflux with fractionation, and 3) complete reflux with fractionation. Where the means to remove HCl from the refluxing crude TDI includes fractionation, HCl removal is increased by affecting the solubility equilibrium of gaseous HCl in the refluxing vapor. Fractionating refluxing vapor increases separation of gaseous HCl from refluxing vapor. Where the means of removal of HCl from refluxing crude TDI includes a partial reflux, HCl removal is increased by a gradual loss of refluxing vapor.

For the purposes of the present invention, fractionation is defined as distillation wherein a rising vapor and a descending liquid are brought into counter-current flow. Hawley's Condensed Chemical Dictionary, 11th ed., p. 540 (1987). For example, in the laboratory, a simple distillation is one wherein a relatively wide condenser having smooth sides and no packing is used to condense rising vapor. In contrast, a fractional distillation can be done using the same condenser after first filling the condenser with a packing material, for example, glass beads. The rising vapor is forced into contact with descending condensate and a more thorough separation of the vapor components is thereby accomplished.

Refluxing, either partial or complete, with fractionation, or partial refluxing, of crude TDI, should continue until bottoms produced from the crude TDI are desirably reactive. Preferably, this treatment is continued until sufficient hydrolyzable chloride is removed from the crude TDI such that, after distillation, the bottoms have a hydrolyzable chloride concentration of less than 1200 ppm hydrolyzable chloride, preferably less than 1000 ppm hydrolyzable chloride, and more preferably less than 750 ppm hydrolyzable chloride.

Complete reflux and fractionation of an admixture of crude TDI with a solvent is a preferred embodiment of the process of the first aspect of this invention. Refluxing the admixture with fractionation causes effective separation of HCl from the refluxing vapors sufficient to produce crude TDI with significantly reduced hydrolyzable chloride. The reduced hydrolyzable chloride crude TDI of this invention is distilled to produce reduced hydrolyzable chloride TDI monomer and also distillation bottoms which are preferably both reactive and viscosity-stable.

In another embodiment of the process of the first aspect of the present invention, crude TDI is not admixed with a solvent, but instead is subjected to a complete reflux and fractionation without solvent. Care should be exercised that the temperatures used to cause reflux and fractionation do not also cause undesirable side reactions.

In still another embodiment of the process of the first aspect, crude TDI, either with or without admixture with a solvent, is subjected to a partial reflux. The removal of uncondensed vapor serves to effect removal of sufficient HCl from crude TDI to produce reactive TDI distillation bottoms. A combination of partial refluxing and fractionation of crude TDI, with or without admixture of the crude TDI with a solvent, is also an embodiment of the present invention.

The first aspect of the present invention is a process for removing hydrolyzable chloride from crude TDI. Any crude TDI is suitable for use in this process. The crude TDI used with this process is preferably formed by the phosgenation of toluene diamine to form crude TDI as described above. However, crude TDI from any source is suitable.

A crude TDI solvent suitable for use with the first aspect can be selected by evaluation of two criteria. First, the solvent should dissolve or suspend crude toluene diisocyanate. Second, the solvent should be an effective means to remove HCl from crude TDI when refluxed, with or without fractionation, with TDI as a solvent admixture. That is, the solvent should be sufficiently compatible with HCl such that the solvent will remove HCl from crude TDI. However, the solvent should not be so highly compatible that, once admixed, the solvent and HCl cannot be separated. Chlorobenzene and ortho-dichlorobenzene are examples of effective solvents for use with this embodiment.

Crude TDI is distilled to prepare TDI monomer and distillation bottoms. For the first aspect of this invention, the crude TDI is preferably distilled by means of a falling film evaporator, but can be distilled in any manner. Both the TDI monomer and distillation bottoms prepared by the process of the first aspect of this invention have reduced hydrolyzable chloride compared to those prepared by distillation of conventional (untreated) crude TDI. In the second aspect of the present invention, TDI distillation bottoms, either prepared conventionally or by the process of the first aspect of the present invention, which are too high in hydrolyzable chlorides to be viscosity-stable and reactive, are treated to reduce hydrolyzable chlorides to a levels such that the bottoms are reactive and viscosity-stable. If the bottoms are released from the still immediately after separation of the purified toluene diisocyanate (i.e., the distillation overheads), it is generally a black tarry liquid. However, when bottoms which are less than 20 weight percent toluene diisocyanate are allowed to cool to 35°C, the bottoms solidify to a pitch-like solid which breaks upon impact but which flows over time. Such cooling delays irreversible hardening such as that typically observed within 6 hours at 150°C to 250°C. Because the hardening occurs even at low temperatures, delays between production of the bottoms and its treatment as provided herein are preferably avoided. Optionally, the TDI bottoms can be dissolved in an organic solvent, such as an aromatic solvent including, for example, orthodichlorobenzene, monochlorobenzene, chlorobenzene, toluene, nitrobenzene, anisole, xylene and mixtures thereof, shortly after their generation. Since the process of the second aspect of the present invention includes heating the TDI bottoms in contact with a material suitable for purging or entraining chloride-containing vapors, dissolution of the TDI bottoms can be in such a material, e.g., in an organic solvent. This predissolution enables processing to complete implementation of the process of the second aspect to be delayed indefinitely, but preferably not more than three days following generation of the bottoms. In one means of employing the process of the second aspect of the present invention the formation of the bottoms and their dissolution in solvent are performed by one party, for example, a TDI manufacturer, and the final treatment of the bottoms to complete the process is performed by another party, for example, a contractor.

In the practice of the second aspect the TDI bottoms starting material is heated in contact with a material which is suitable for purging chloride-containing vapors. This means that the material associates, either physically, chemically, or a combination thereof, with the chloride compounds present in the TDI bottoms, e.g., the hydrolyzable chloride compounds, but not with significant amounts of other components present in the TDI bottoms. Where there is a chemical reaction of the material with the chloride-containing vapors, such reaction is preferably reversible. Thus, the vapors are "purged" under specific reaction conditions. This purging allows separation of the material from the TDI bottoms and the resultant removal of the chloride-containing compounds.

One way to do this is to heat the TDI bottoms dissolved in a solvent which is suitable for purging the chloride-containing vapors. As noted above, such dissolution preferably occurs soon after generation of the bottoms. It is preferred that this solvent is an organic solvent in which the TDI bottoms can be at least 90 percent dissolved, and more preferably 100 percent dissolved. The solvent can be selected from a wide range of organic and aromatic solvents, including but not limited to aromatic solvents such as orthodichlorobenzene (ODCB), chlorobenzene and other chlorinated solvents; toluene, nitrobenzene; anisole; xylene and mixtures thereof. Other aprotic, preferably weakly nucleophilic solvents can also preferably be employed. It is preferred that the solvent be selected such that its boiling point is above 40°C at atmospheric pressure, more preferably from 40°C to 260°C at atmospheric pressure, and most preferably from 150°C to 260°C.

The TDI bottoms/solvent solution is then heated to a temperature to distill the solution and thereby separate the solvent carrying the chloride-containing compounds from the bottoms, i.e., above the boiling point of the selected solvent. For example, in the case of using an aromatic chlorohydrocarbon such as orthodichlorobenzene (ODCB), the temperature is preferably from 180°C to 200°C. The temperature used will vary with the solvent. A solvent with a higher boiling point will require either a higher processing temperature or else a lowered pressure, whereas a solvent having a lower boiling point will process at either a lower temperature or a higher pressure. The heating separates the solvent as a vapor and, if the TDI residues are going to be blended with another isocyanate product, is preferably continued until the TDI bottoms reach at least 10 percent by weight concentration in the solvent, more preferably at least 50 percent, and most preferably at least 75 percent. If the TDI bottoms are not going to be blended with another isocyanate product, they are preferably distilled until they represent at least 95 percent, more preferably at least 99 percent, and most preferably at least 99.9 percent by weight in the solvent.

Heating can be carried out either batch-wise or continuously, as a simple distillation or a partial or complete reflux, with or without fractionation. Removal of the solvent results in removal of significant amounts of hydrolyzable chlorides and resulting viscosity-stabilization. Solvent stripping can also be used to augment the removal of solvent, and can be effectively accomplished preferably at low pressure. For example, vacuum stripping can be employed to increase the amount of hydrolyzable chlorides removed from the bottoms.

5

In another embodiment of the process of the second aspect of the present invention, the TDI bottoms are sparged with a gas which associates with the chloride-containing vapors but not significantly with other components of the TDI bottoms. Nitrogen or hydrogen chloride, for example, are preferred selections. This sparging is preferably done with the bottoms dissolved in an organic solvent such as described hereinabove, and can be done before, during or after heating in contact with a material suitable for purging or entraining the chloride containing vapors. It is to be understood that this is treatment of the TDI bottoms only, and not treatment of the crude TDI stream prior to the conventional distillation which separates a "purified" commercial TDI product from the TDI distillation residue. For example, when orthodichlorobenzene is selected, it is preferred that the heating takes place in a range of from 50°C to 230°C, more preferably from 140°C to 220°C, and most preferably from 160°C to 200°C. Total time can vary preferably from 5 minutes to 210 minutes. Other solvents can require different temperatures or times. At the most preferred temperature range the residence time is preferably from 1 to 3 hours. Those skilled in the art will know to balance temperature and residence time to achieve acceptable or desirable processing economics and to optimize chloride reduction within the range of the selected processing condition ranges.

In preparing the treated TDI bottoms of the second aspect of the present invention, either for use alone or as a component of a treated TDI bottoms/polyisocyanate blend, it is desirable that all solvent used in the treatment of the present invention be removed prior to reaction to prepare a polyurethane. Preferably, the solvent is removed by heating the TDI bottoms or TDI bottoms/polyisocyanate blend using low pressure methods known to those skilled in the art, e.g., vacuum stripping. If necessary, however, a second distillation can be performed following blending to remove any remaining solvent. There is preferably no more than trace amounts of solvent present in the final, viscosity-stabilized product, preferably less than 1 percent by weight, more preferably less than 0.008 percent by weight. This can be determined by means of standard gas chromatography using methods known to and conventionally used by those skilled in the art.

Following removal of the solvent, the treated TDI bottoms or treated TDI bottoms/polyisocyanate blend product is preferably cooled. This cooling can be done from 40 to 70°C, but is preferably done at 50°C over a very short time period, preferably in less than 60 minutes, more preferably less than 30 minutes, and most preferably less than 5 minutes. The product is then preferably stored at 50°C for from 1 to 14 days, more preferably 7 days, and then allowed to cool to 25°C. This allows reequilibrium of the viscosity-affecting materials. A cooling step can also be done if desired. Following preparation of the treated TDI bottoms product, it is ready for use in preparing a final product, such as a polyurethane, polyurea, or polyisocyanurate product or a prepolymer useful to prepare a final product, by reacting it with an active hydrogen compound.

Both of the aspects of the present invention involve reducing hydrolyzable chlorides. Determination of hydrolyzable chloride can be done according to the procedure described below. Hydrolyzable chloride can be determined by liberating ionic chloride from the isocyanate and titrating the resulting chloride ion concentration with, for example, silver nitrate. An admixture of a known weight of isocyanate and a mixture of 50 parts by weight toluene, 50 parts by weight methanol, and 6.5 parts by weight pyrrolidine is first prepared in a quantity sufficient to react with the diisocyanate and dissolve the products of the reaction therewith. A solution is formed by stirring the admixture for four minutes. A quantity of 4 ml concentrated nitric acid (85 weight percent), sufficient to maintain activity of silver/silver chloride electrodes used to determine the end point of titration, is added. The solution is stirred for one additional minute. The electrodes are inserted and titration with a dilute solution (e.g., 0.025 Normal (N)) of silver nitrate is begun. Titration is ended when an inflection point is determined. The concentration of hydrolyzable chloride is calculated from the amount of silver nitrate consumed.

Both crude TDI and the distillation bottoms generally contain acidic materials left over from the phosgenation process. The term "acid", as used herein, refers to these contaminants as well as to free HCl and/or labile covalently bonded chlorides present in the isocyanate, such as carbamoyl chlorides and others, that respond as acids in standard analytical tests.

The acid content or level, of crude TDI and TDI distillation bottoms, is readily determined by standard analytical tests such as ASTM D-4667-87 or other tests for acidity. These tests generally involve heating the isocyanate in a solution of mixed alcohols or toluene and methanol, and titrating the resulting mixture with dilute potassium hydroxide. Acidity is expressed as weight percent HCl. The TDI distillation bottoms of either aspect of this invention, are preferably reactive. For the purposes of this invention, the term "reactive" is defined as the ability of the TDI bottoms to advantageously react with a compound containing an active hydrogen containing group. Any suitable organic compound containing an active hydrogen containing group, as determined by the Zerewitinoff method, can be used for reaction with the distillation bottoms or blends thereof with other polyisocyanates. Active hydrogen compounds are compounds having hydrogen-containing functional groups which will react with an isocyanate group. The Zerewitinoff test described by Kohler in the Journal of the American Chemical Society, Vol. 49, page 3181 (1927) predicts the tendency of a hydrogen- containing group to react with isocyanates.

When PMDI materials are blended with the TDI distillation bottoms of either aspect of the present invention, these blends are preferably at least as reactive as the starting PMDI materials. This reactivity can be determined by measuring the time from mixing of an isocyanate or an admixture of isocyanates with an active hydrogen compound until specific phenomena are observed in a forming polyurethane or polyisocyanurate foam. Materials containing a high concentration of hydrolyzable chloride, some or all of which can be in the form of acidity, tend to either not foam at all, or to fail to timely cure, with the result that they remain tacky. The measurements of foam forming ability include: a) Cream time: the time in seconds from mixing until foaming begins, determined by observing when gas first begins to separate from the admixture; b) Gel time: the time in seconds from mixing until the foaming admixture first begins to produce "strings" adhering to a wooden spatula quickly inserted and removed from the foaming admixture; c) Tack-free time: the time in seconds from mixing until the foam surface loses its sticky quality. Reactive bottoms are those that can be readily made into foam with suitably fast cream, gel, and tack-free times.

Active hydrogen components most commonly used in polyurethane production are those compounds having at least two hydroxyl groups. Those compounds are referred to herein as polyols. Representatives of suitable polyols are generally known and are described in such publications as High Polymers, Vol. XVI, "Polyurethanes, Chemistry and Technology" by Saunders and Frisch, Interscience Publishers, New York, Vol. I, pp. 32-42, 44-54 (1962) and Vol. II, pp. 5-6,198-199 (1964); Organic Polymer Chemistry by K. J. Saunders, Chapman and Hall, London, pp. 323-325 (1973); and Developments in Polyurethanes, Vol. I, J. M. Burst, ed., Applied Science Publishers, pp. 1-76 (1978).

Typical polyols include polyester polyols, polyester amide polyols, and polyether polyols having at least two hydroxyl groups. Polyethers and polyesters having hydroxyl terminated chains are preferred for use as relatively high molecular weight active hydrogen containing compounds for use in forming polyurethanes in particular. Examples of polyols also include hydroxy functional acrylic polymers, hydroxyl-containing epoxy resins, polyhydroxy terminated polyurethane polymers, polyhydroxyl-containing phosphorus compounds and alkylene oxide adducts of polyhydric thioethers, including polythioethers and acetals, such as polyacetals. Aminated polyols can also be used.

The amount of TDI monomer present in the bottoms is a function of the producing unit's ability to handle the bottoms as their viscosity increases with time. Units which can quickly dispose of the bottoms are able to distill more TDI monomer from the crude TDI than units which must hold the bottoms for a significant time period prior to disposal. Bottoms produced by the process of either aspect of this invention contain less than 50 percent, preferably less than 25 percent of TDI monomer.

The low hydrolyzable chloride TDI bottoms of either aspect of this invention are desirably reactive and viscosity-stable. Untreated TDI distillation bottoms quickly increase in viscosity. Advantageously, the bottoms produced by the process of the present invention will, by comparison, increase in viscosity more slowly and/or to a lesser extent than untreated bottoms. The viscosity stability of the distillation bottoms can be determined by evaluating changes in viscosity beginning immediately after they are prepared. Distillation bottoms can be considered viscosity-stable if their viscosity in centipoise (pascal-second), as measured by ASTM-D445-88, increases by no more than 100 percent within the first 3 hours, preferably within the first 4 hours, and more preferably within the first 5 hours of the conclusion of the distillation from which the bottoms resulted, when the bottoms contain 25 percent TDI monomer and are stored at 100°C.

The TDI distillation bottoms prepared using the process of either aspect of the present invention are suitably used without dilution, for instance, to prepare a foam. However, the bottoms can be blended with another polyisocyanate different from the distillation bottoms in an amount sufficient to achieve a preselected viscosity and/or a desired reactivity as evidenced by the ability to form a polymeric foam and the qualities of the foam formed. The polyisocyanate used for dilution is suitably any, preferably liquid, organic isocyanate compound having an average of more than one isocyanate group per molecule. The polyisocyanate is suitably crude or distilled, but preferably has a viscosity less than that of the TDI distillation bottoms. Such polyisocyanate compounds are well known and readily available commercially.

Examples of suitable polyisocyanates include aromatic, aliphatic and cycloaliphatic polyisocyanates and combinations thereof. Representative polyisocyanates include diisocyanates such as m- phenylene diisocyanate, toluene-2,4-diisocyanate, toluene-2,6-diisocyanate, hexamethylene-diisocyanate, tetra-methylene-diisocyanate, cyclohexane-1,4- diisocyanate, hexahydrotoluene diisocyanate and isomers thereof, 1-methoxyphenyl-2,4-diisocyanate, diphenylmethane-4,4'-diisocyanate, diphenylmethane-2,4'-diisocyanate, 3,3'-dimethyl-4,4'-biphenyl diisocyanate, 3,3'-dimethyl-diphenyl-methane-4,4'-diisocyanate and the like; triisocyanates such as 4,4',4''-triphenylmethane triisocyanate, and toluene-2,4,6-triisocyanate; tetraisocyanates such as 4,4'-dimethyldiphenyl-methane-2,2',5,5'-tetraisocyanate, 4,4'-dicyclohexane- diisocyanate, isophorone diisocyanate, and isomers of each; other polyisocyanates such as polyphenylisocyanate; and mixtures thereof. TDI, diphenylmethane-4,4'-diisocyanate, diphenylmethane- 2,4'-diisocyanate and PMDI materials are preferred because of their availability and properties. Mixtures of polyisocyanate components are particularly preferred.

PMDI materials are preferred for use in the practice of either embodiment of the present invention. PMDI materials are mixtures containing from 35 to 85, preferably from 65 to 75, percent by weight of methylene diphenyl diisocyanate, the remainder of the mixture being closely related polyisocyanates of higher molecular weight and functionality greater than two. They are well-known compositions, and are commercially prepared by phosgenation of mixtures of the corresponding methylene-bridged polyphenyl polyamines.

Blends of the bottoms of either aspect of the invention are composed of from 1 to 99 percent by weight TDI bottoms, and the remainder polyisocyanate. From 90 to 99 weight percent bottoms based on total weight of a blend are suitably used; however, preferably, the bottoms blend contains at least 10, more preferably from 20 to 75, and most preferably from 20 to 40 weight percent bottoms based on total weight of the blend. The relative proportions of bottoms and polyisocyanate are generally selected to achieve a preselected viscosity and preselected properties in resulting products. Advantageously, the relative proportions of bottoms and polyisocyanate are selected to achieve a viscosity suitable for a use of the blend.

Advantageously, for use in making polymer foams, the blend has a viscosity of less than 10,000 centipoise (10 Pa•s), preferably from 30 to 3,000 centipoise (0.03 to 3 Pa•s), more preferably from 40 to 2,500 centipoise (0.04 to 2.5 Pa•s). When a blend is to be used for a specific application, the viscosity is most preferably preselected for convenience in preparing that type of material by processes known to those skilled in the art. For instance, in the case of insulative polyurethane foams, viscosity is generally preferably from 200 to 3,000 centipoise (0.2 to 3 Pa•s).

When bottoms are to be used in applications sensitive to specific levels of acidity, care should be exercised that only those distillation bottoms with an appropriate acidity concentration are used. Where an application requires such an isocyanate, bottoms having acidity of no more than 500 ppm are preferably blended with isocyanates having less acidity such that the blend has less than 400 ppm, preferably less than 300 ppm, acidity.

Blends of the distillation bottoms and liquid polyisocyanates are suitably used to make polyisocyanurate, polyurethane, polyurea, and polyurethane-polyurea polymers and mixtures thereof. The polymers suitably take the form of products such as flexible or rigid foams, adhesives, binders and the like. Polyisocyanurate foams are foams formed using a ratio of isocyanate groups to active hydrogen groups of at least 1.3, preferably, in the presence of trimerization catalysts. Polyurethane foams are formed when little trimerization of isocyanate takes place, and polymer formation is primarily the reaction of active hydrogen groups of an active hydrogen component with isocyanate groups of a polyisocyanate component.

The distillation bottoms produced by the process of either aspect of the present invention are advantageously reacted with active hydrogen compounds in the presence of a blowing agent. Any blowing agent or mixture thereof is suitable for this use. Suitable blowing agents include inorganic blowing agents such as water, organic blowing agents which are volatile at reaction temperatures and dissolved inert gases. Suitable organic blowing agents include acetone; ethyl acetate; methanol; ethanol; halogen-substituted alkanes such as methylene chloride, chloroform, ethylidene chloride, vinylidene chloride, monofluorotrichloromethane, and chlorodifluoromethane, dichlorodifluoromethane; butane; hexane; heptane; and diethyl ether. Gases inert to the starting components such as nitrogen, air, carbon dioxide are also useful blowing agents. Compounds, such as azides, which decompose at suitable temperatures to produce gases such as nitrogen are also useful. Preferred blowing agents are compounds which boil between -50 and 100°C, more preferably between 0 and 50°C.

The amount of blowing agent employed is not critical, but is preferably sufficient to foam the reaction mixture. The amount will vary with factors such as the density desired in a foamed product.

Water is a useful blowing agent for use in the practice of the preparing foams from formulations including reactive TDI bottoms. In addition to generating carbon dioxide gas for foaming, water reacts quickly with polyisocyanate components, thus contributing to early polymer strength needed for gas retention. Generally, when water is used, it is present in proportions of from 1.5 to 8 weight percent of water based on total weight of active hydrogen components. Other blowing agents can be used with water.

Rigid polyisocyanurate and polyurethane foams prepared from the distillation bottoms of either embodiment of the present invention are particularly useful. Those skilled in the art of preparing such foams can readily use reactive TDI distillation bottoms, or blends thereof, with other polyisocyanates, to prepare the foams.

Processes known to those skilled in the art can suitably be used to prepare a rigid polyisocyanurate foam using reactive TDI bottoms, or blends thereof, with other polyisocyanates. Preferably, the reactive distillation bottoms, or blends of thereof, are reacted with a polyfunctional active hydrogen compound, in the presence of a catalyst, which catalyzes the formation of isocyanurates, and a blowing agent suitable for forming foams having preselected physical properties.

Suitable catalysts are those which catalyze the formation of isocyanurates such as those mentioned in High Polymers, Vol. XVI, "Polyurethanes, Chemistry and Technology" by Saunders and Frisch, Interscience Publishers, New York, part 1, pp. 94-97 (1962). Such catalysts are referred to herein as trimerization catalysts. Examples of these catalysts include aliphatic and aromatic tertiary amine compounds, organometallic compounds, alkali metal salts of carboxylic acids, phenols and symmetrical triazine derivatives. Preferred catalysts are potassium salts of carboxylic acids such as potassium octoate and tertiary amines such as, for instance, 2,4,6-tris(dimethyl aminomethyl) phenol.

The following examples and comparative examples serve to illustrate the present invention. Comparative examples are presented for comparison purposes only, and do not represent embodiments of the present invention.

EXAMPLE 1

A complete reflux and fractionation of crude TDI was carried out by placing 600 ml of a 10 percent crude TDI and 90 percent ortho-dichlorobenzene solution in a 1000 ml three-necked flask. A one-foot (30.5 cm) vacuum jacketed Vigreux column was placed on the central neck of the flask for fractionation. A water cooled West condenser was placed on top of the Vigreux column for reflux. The solution was heated to 185°C in 18 minutes. The temperature was maintained at 186°C for the remainder of the experiment. Overhead vapors are condensed in the West column and returned to the flask through the Vigreux column. Samples are taken at 8 minute intervals and analyzed for hydrolyzable chloride. At the beginning of this experiment, the hydrolyzable chloride concentration of the crude TDI, corrected for dilution, was 12,093 ppm. After 72 minutes, the concentration of hydrolyzable chloride was 916 ppm.

EXAMPLE 2

A partial reflux was carried out by repeating the procedure of Example 1 substantially identically except that overhead vapors are condensed and removed from the system. At the beginning of this experiment, the crude TDI has a hydrolyzable chloride concentration, corrected for dilution, of 12,021 ppm. After 72 minutes, the concentration of hydrolyzable chloride was 831 ppm.

COMPARATIVE EXAMPLE 3

A complete reflux with no fractionation was carried out by repeating the procedure of Example 1 substantially identically except that the Vigreux column was not used. At the beginning of this experiment, the crude TDI had a hydrolyzable chloride concentration, corrected for dilution, of 11,905 ppm. After 72 minutes, the concentration of hydrolyzable chloride was 1,565 ppm.

EXAMPLE 4

Crude TDI prepared according to Example 1 was distilled to form TDI monomer and distillation bottoms, the bottoms containing 22.3 percent TDI monomer. The distillation bottoms were stored at 100°C and viscosity was measured periodically. Viscosity growth is shown in Table 1.

COMPARATIVE EXAMPLE 5

The materials and procedures of Example 4 are repeated substantially identically except that untreated distillation bottoms were used in place of the treated distillation bottoms, and the bottoms contain 25.1 percent TDI monomer. Viscosity growth is shown in Table 1.

TABLE 1

| Minutes Following Preparation 100°C | Example 4 Centipoise (Pascal•second) | Comparative Example 5 Centipoise (Pascal•second) |
|---|---|---|
| 0 | 105.5 (0.105) | 35.8 (0.004) |
| 10 | 136.9 (0.137) | 45.1 (0.045) |
| 20 | 140.3 (0.140) | 46.8 (0.047) |
| 40 | 150.0 (0.150) | 51.1 (0.051) |
| 102 | 174.5 (0.174) | 68.5 (0.069) |
| 197 | 193.5 (0. 194) | 96.1 (0.096) |
| 300 | 208.9 (0.209) | 152 (0.152) |
| 644 | 239.8 (0.240) | 956.8 (0.96) |
| 1432 | 287.3 (0.287) | ** |
| ** indicates that the material would not flow through the viscometer | | |

EXAMPLE 6

A foam was prepared by mixing 100 parts of an "A" component composed of 75 parts PMDI and 25 parts TDI bottoms bottoms as prepared in Example 4, with 118 parts of a "B" component prepared using a polyurethane foam formulation comprising polyether and amine polyols, catalysts, surfactants, and water and trichlorofluoromethane as blowing agents. This foam had a cream time of 3 seconds, a gel time of 23 seconds, and a tack-free time of 30 seconds.

COMPARATIVE EXAMPLE 7

A foam was prepared using the formulation and process of Example 6 except that 100 parts of PMDI and no TDI distillation bottoms are used to prepare the "A" component. This foam had a cream time of 4 seconds, a gel time of 23 seconds, and a tack-free time of 33 seconds.

COMPARATIVE EXAMPLE 8

An attempt was made to prepare a foam using the formulation and process of Example 6 except that the "A" component was prepared using 25 parts of untreated TDI bottoms and 75 parts PMDI. The reaction mixture did not produce sufficient heat to vaporize the blowing agent and therefore did not foam.

EXAMPLE 9

82.0 g of TDI distillation residue resulting from commercial preparation of TDI, containing 4,000 ppm chloride, was dissolved in 394.5 g of orthodichlorobenzene (ODCB). This 80 percent ODCB/20 percent TDI bottoms solution was heated to distill it with concurrent solvent removal at 190°C. The distillation was stopped when the ODCB concentration was 20 percent. Polymeric methylene diphenyldiisocyanate (PMDI) having a viscosity of 42 centipoise (0.042 Pa.s) was blended with the treated TDI bottoms/ODCB solution. The blend was prepared such that the final treated TDI bottoms/PMDI blend product was composed of 25 percent treated TDI bottoms and 75 percent PMDI. In order to remove the remaining solvent, the resultant mixture was heated at 150°C and 8 mm Hg (1 kPa) pressure.

After 30 minutes of solvent removal, the treated TDI bottoms/PMDI mixture was allowed to cool to 50°C. This treated TDI bottoms/PMDI blend product was stored at 50°C for 3 days to allow reequilibration of viscosity, and then allowed to cool to 25°C. The blended product had a hydrolyzable chloride level of 237 ppm chloride. A urethane reactivity test gives a relative cream time of 45 seconds. The viscosity growth rate is as shown in Table 2.

10

TABLE 2

| TIME (Days) | VISCOSITY at 25˚C centapoise/Pa•s | INSTANTANEOUS VISCOSITY GROWTH RATE at 25˚C (% Per Month) | VISCOSITY at 50˚C centapoise/Pa•s | INSTANTANEOUS VISCOSITY GROWTH RATE at 50˚C (% Per Month) |
|---|---|---|---|---|
| 0 | 304/0.304 | n/a | 304/0.304 | n/a |
| 14 | 332/0.332 | 19.7 | 379/0.379 | 52.9 |
| 28 | 329/0.329 | -1.9 | 414/0.414 | 19.8 |
| 42 | 344/0.344 | 9.8 | 438/0.438 | 12.4 |
| 56 | 351/0.351 | 4.4 | 480/0.48 | 20.5 |
| 90 | 365/0.365 | 3.5 | 573/0.573 | 17.1 |
| 119 | 376/0.376 | 3.1 | 699/0.699 | 22.7 |
| 154 | 377/0.377 | 0.2 | 785/0.785 | 10.5 |

n/a indicates not applicable, i.e., row shows only starting viscosities after reequilibration (at 3 days after mixing).

11

EXAMPLE 10

TDI bottoms (121.0 g) from a TDI plant utilizing a thin film evaporator was admixed with 362.4 g of PMDI. The viscosity of the PMDI was 40 centipoise (0.040 Pa.s). The resultant mixture had a viscosity of 160 cps (0.160 Pa.s). The blend was poured into a 1,000-mL, round-bottom flask equipped with a nitrogen sparger, magnetic stirrer, heating mantle and stirrer. Nitrogen was then sparged though the blend at 350 mL/min. and the mixture was heated.

After 20 minutes, a temperature of 170°C was attained and held for 3 hours. The blend was quickly cooled by immersion of the flask into an ice-water bath. Acidity was measured at 147 ppm. Aliquots of the treated TDI bottoms:PMDI blend were placed in 4-dram (16 g) vials, sealed and stored at 25°C and 50°C. The solutions were allowed to reequilibrate for 3 days before viscosity measurements are taken. Viscosity measurements are shown in Table 3.

Table 3

| TIME (Days) | VISCOSITY at 25°C centapoise/Pa•s | INSTANTANEOUS VISCOSITY GROWTH RATE at 25°C (% Per Month) | VISCOSITY at 50°C centapoise/Pas | INSTANTANEOUS VISCOSITY GROWTH RATE at 50°C (% Per Month) |
|---|---|---|---|---|
| 0 | 309/0.309 | n/a | 309/0.309 | n/a |
| 14 | 327/0.327 | 12.5 | 352/0.352 | 29.8 |
| 28 | 338/0.338 | 7.2 | 395/0.395 | 26.2 |
| 49 | 343/0.343 | 2.1 | 449/0.449 | 19.5 |
| 83 | 355/0.355 | 3.1 | 582/0.582 | 26.1 |
| 121 | 359/0.359 | 0.9 | 804/0.804 | 30.1 |
| 162 | 371/0.371 | 2.4 | 1278/1.28 | 43.1 |
| 191 | 380/0.38 | 2.5 | -- | -- |
| n/a indicates not applicable, i.e.,row shows only starting viscosities after reequilibration (at 3 days after mixing). --denotes no data available. | | | | |

COMPARATIVE EXAMPLE 11

A treated TDI bottoms/PMDI blend was prepared using the method and amounts of Example 9, except that the final solvent removal step was done prior to blending the treated TDI bottoms and PMDI, rather than after the blending as in Example 9.

96.8 g of untreated TDI bottoms was blended with 290.1 g of PMDI having a viscosity of 42 cps (0.042 Pa.s). The hydrolyzable chloride level was calculated as 1,157 ppm chloride. The urethane reactivity test gave a relative gel time of 53 seconds. The viscosity growth rate is as shown in Table 4.

TABLE 4

| TIME (Days) | VISCOSITY at 25°C centapoise/Pa•s | INSTANTANEOUS VISCOSITY GROWTH RATE at 25°C (% Per Month) | VISCOSITY at 50°C centapoise/Pa•s | INSTANTANEOUS VISCOSITY GROWTH RATE at 50°C (% Per Month) |
|---|---|---|---|---|
| 0 | 278/0.278 | n/a | 278/0.278 | n/a |
| 14 | 313/0.313 | 27.0 | 609/0.609 | 255.1 |
| 28 | 331/0.331 | 12.3 | 1882/1.88 | 447.9 |
| 42 | 351/0.351 | 12.9 | 4608/4.61 | 310.4 |
| 56 | 367/0.367 | 9.8 | 18169/18 | 630.6 |
| 90 | -- | -- | -- | -- |
| 119 | 442/0.442 | 9.7 | -- | -- |
| 154 | 519/0.519 | 14.9 | -- | -- |
| n/a denotes not applicable,i.e., row shows only starting viscosities after reequilibration (at 3 days after mixing). --denotes no data available. | | | | |

**Claims**

1. A process for reducing hydrolyzable chloride content of toluene diisocyanate distillation bottoms comprising subjecting crude toluene diisocyanate prior to distillation to partial reflux with or without fractionation, or to complete reflux with fractionation, to reduce the hydrolyzable chloride content thereof and/or contacting the toluene diisocyanate bottoms with a material suitable to purge chloride-containing vapors to remove said vapors from said bottoms.

2. A process as claimed in Claim 1, wherein the crude toluene diisocyanate prior to distillation is subjected to partial reflux with or without fractionation.

3. A process as claimed in Claim 1, wherein the crude toluene diisocyanate prior to distillation is subjected to complete reflux with fractionation.

4. A process as claimed in any one of the preceding claims, wherein the crude toluene diisocyanate is admixed with a solvent prior to said refluxing.

5. A process as claimed in Claim 4, wherein the solvent is chlorobenzene or orthodichlorobenzene.

6. A process as claimed in any one of the preceding claims, wherein the crude toluene is refluxed at a temperature of from 180°C to 195°C.

7. A process as claimed in any one of the preceding claims, wherein said refluxing is continued for a period of time sufficient to reduce hydrolyzable chloride in the crude toluene diisocyanate to a concentration such that, after distillation of toluene diisocyanate monomer, the toluene diisocyanate distillation bottoms have a hydrolyzable chloride concentration of less than 1000 ppm.

8. A process as claimed in any one of the preceding claims, wherein the crude toluene diisocyanate has been formed by phosgenation of toluene diamine.

9. A process as claimed in any one of the preceding claims, which comprises contacting the toluene diisocyanate bottoms with a material suitable to purge chloride-containing vapors to remove said vapors from said bottoms.

10. A process as claimed in Claim 9, wherein the said contacting comprises heating in an aromatic solvent.

11. A process as claimed in Claim 10, wherein the solvent is selected from orthodichlorobenzene, toluene, benzene, nitrobenzene, anisole, xylene, monochlorobenzene, and mixtures thereof.

12. A process as claimed in Claim 10 or Claim 11, wherein the heating is at a temperature from 150°C to 230°C and for a time period of from 5 to 210 minutes.

13. A process as claimed in any one of Claims 9 to 12, wherein said contacting comprises sparging with a gas.

14. A process as claimed in Claim 13, wherein said gas is nitrogen or hydrogen chloride.

15. A process as claimed in any one of the preceding claims, wherein the reflux and/or contact is conducted for sufficient time to provide reactive and viscosity stable bottoms.

16. A process as claimed in any one of the preceding claims, wherein the process conditions are such as to yield toluene diisocyanate distillation bottoms having (a) toluene diisocyanate monomer concentration of less than 50 percent, and (b) a viscosity growth rate of no more than 100 percent measured 3 hours after completion of the distillation step when the bottoms contain 25 percent toluene diisocyanate monomer and are stored at 100°C.

16